# EUROPEAN PATENT APPLICATION

(11) **EP 1 930 343 A1**
(43) Date of publication of application: **11.06.2008**
(21) Application number: 06796638.2
(22) Date of filing: 22.08.2006
(51) Int. Cl.: C07K 14/47, C07K 1/113, C07K 1/14, A61K 8/96, A61K 47/42, A61L 27/00

(54) **METHOD FOR PREPARATION OF REDUCED KERATIN, REDUCED CUTICLE PROTEIN OR MIXTURE THEREOF**

(30) Priority: 23.08.2005 JP 2005240840
(71) Applicant: Seiwa Kasei Company, Limited, Higashiosaka-shi Osaka 579-8004 (JP); Yamauchi, Kiyoshi, Kyoto-shi, Kyoto 610-1101 (JP)
(72) Inventor: YAMAUCHI, Kiyoshi, Kyoto-shi, Kyoto 610-1101 (JP)
(74) Representative: Vossius & Partner
(86) International application number: PCT/JP2006/316424
(87) International publication number: WO 2007/023816

(57) **Abstract**

A method for producing a reduced keratin solution, a powder of reduced keratin, a reduced cuticle protein dispersion, an aqueous medium dispersion containing reduced keratin and reduced cuticle protein, or a mixed powder of reduced keratin and reduced cuticle protein, comprising the extraction step of dipping and heating a keratin-containing substance in an aqueous medium containing a reducing agent and a surface active agent, without using a protein denaturing agent, such as urea, to extract the water-soluble component contained in the keratin-containing substance into the aqueous medium.

## Description

### TECHNICAL FIELD

The present invention relates to a method for producing reduced keratin, reduced cuticle protein and the mixture thereof, characterized in that the disulfide bonds in a keratin-containing substance, such as hair, animal hair and feathers, are converted into thiol groups (SH groups) through a reduction or substitution reaction by treating the keratin-containing substance in an aqueous medium in which a reducing agent and a surface active agent are dissolved, without using a protein denaturing agent, such as urea. With this method according to the present invention, keratin and cuticle protein, main ingredients of the keratin-containing substance, are isolated as reduced protein containing numerous cysteine residues, prepared as solutions, dispersions or powders, and used to produce products for various industries, such as films, sheets, fibers, sponges, coating materials, cosmetic materials, medical materials and artificial ivories.

### BACKGROUND ART

Keratin that is present as structural protein in animal tissues, such as hair, animal hair, feathers and horns, has heretofore attracted attention as a material for basic industrial materials, such as films and fibers (Nonpatent Documents 1, 2 and 3). However, keratin is usually insoluble or hardly soluble in solvents. To utilize keratin in substances containing keratin, such as hair, animal hair, feathers and horns, that is, to utilize keratin in keratin-containing substances, it is necessary to unwind the three-dimensional structure of the keratin by fragmenting the keratin molecules (by shortening the molecules) through hydrolysis or by cleaving the disulfide bonds in keratin protein by carrying out oxidation or reduction treatment.

As methods for utilizing keratin in a keratin-containing substance, for example, the following methods are known, which utilize:
oxidized keratin obtained by treating a keratin-containing substance with an oxidant, such as formic acid-water-hydrogen peroxide,
hydrolysate obtained by treating a keratin-containing substance with acid, alkali or enzyme (Patent Documents 1, 2 and 3),
keratin fragments prepared by subjecting a keratin-containing substance to reduction treatment and protein degradation enzyme treatment (Patent Documents 4, 5, 6 and 7),
gel-like modified keratin prepared by the combination of partial oxidation and reduction of hair or the like and the reaction with thioglycolic acid (Patent Document 8),
keratin holding -S-S-CH₂COOH which is obtained by treating hair or the like with a reducing agent under alkaline condition and then by treating the hair or the like with an oxidant under the presence of thioglycolic acid (Patent Document 9),
reduced keratin aqueous solution in which the disulfide bonds of keratin are reduced and cleaved to form thiol groups by using a reducing agent and a protein denaturing agent, such as urea, as necessary components (Patent Documents 10, 11 and 12), and
reduced keratin aqueous solution in which the disulfide bonds of keratin are reduced and cleaved to form thiol groups using three substances, namely, a reducing agent, urea or thiourea aqueous solution and a surface active agent (Patent Documents 4 and 5).

Furthermore, the following methods are also known, which produce:
a keratin derivative that is irreversibly chemically modified by using monoiodoacetic acid, sodium sulfite/sodium tetrathionate or the like to prevent the rebinding of the thiol groups of keratin in keratin aqueous solution (Patent Documents 14 and Nonpatent Document 6),
a substance provided with functions of adsorbing and
removing heavy metal ions in industrial drainage by reducing a keratin-containing substance in an aqueous or organic medium and then by making the substance into contact with a metal salt (Patent Document 15), and
insoluble reduced protein derived from animal cuticle,
   characterized by being obtained using a reducing agent and a protein denaturing agent, such as urea or thiourea (Patent Documents 16 and 17).
   Patent Document 1: JP 54-89987A
   Patent Document 2: JP 60-27680B
   Patent Document 3: JP 3-11099A
   Patent Document 4: JP 55-38358B
   Patent Document 5: JP 61-183298A
   Patent Document 6: JP 3-11099A
   Patent Document 7: JP 6-116300A
   Patent Document 8: US6124265(2000)
   Patent Document 9: US5763583(1998)
   Patent Document 10: Japanese Patent No. 3283302
   Patent Document 11: JP 63-301809A
   Patent Document 12: JP 8-175938A
   Patent Document 13: Japanese Patent No. 2946491
   Patent Document 14: JP 57-23631A
   Patent Document 15: JP 53-23999A
   Patent Document 16: JP 6-336499A
   Patent Document 17: JP 10-291999A
   Nonpatent Document 1: Kiyoshi Yamauchi, "Kobunshi: Tenbo", Vol. 50, pp. 240-243 (2001)
   Nonpatent Document 2: Kiyoshi Yamauchi, "Kagaku to Kougyo: Lecture", Vol. 78, pp. 384-392 (2004)
   Nonpatent Document 3: Kunio Shirai, Yasuhiro Ishii, "Hikakukagaku", Vol. 44, pp. 1-10 (1998)
   Nonpatent Document 4: K. Yamauchi, et al., "J. Biomed. Mat. Res.", 31, 439-444 (1996)
   Nonpatent Document 5: Toshihiro Fujii, et al., "Kobunshi Ronbunshu", Vol. 60, pp. 354-358 (2003)
   Nonpatent Document 6: R.S. Asquith, et al., "Chemistry of Natural Protein Fibers", Prenum Press (1977), p.193

### DISCLOSURE OF THE INVENTION

### PROBLEMS TO BE SOLVED BY THE INVENTION

Oxidized keratin obtained by oxidizing a keratin-containing substance is widely known. However, since the thiol groups of the oxidized keratin have been changed to sulfonic acid groups or the like, the oxidized keratin cannot exhibit the oxidation polymerization property peculiar to keratin. The method in which a keratin-containing substance, such as hair, is treated using a reducing agent under alkaline condition and extracted (Patent Document 9) is a method performed without using a protein denaturing agent, such as urea. However, the SH groups of the cysteine residues are chemically modified using thioglycolic acid or the like, and the oxidative cross-linking property of the keratin is lost. Furthermore, the hydrolysis of protein occurs inevitably while the substance is treated under alkaline condition. It is thus inevitable that the molecules are shortened.

On the other hand, as methods for preparing reduced keratin containing numerous cysteine residues, for example, the following methods are known: a method in which keratin is reduced and made soluble in a solvent primarily consisting of water under alkaline condition and under the presence of a solubilizing agent comprising a reducing agent, such as thiol, and a large amount of protein denaturing agent, such as urea (Patent Documents 10, 11 and 12), and a method for producing a stable reduced keratin aqueous solution using a surface active agent concurrently under neutral condition (Patent Document 11 and Nonpatent Documents 4 and 5).

However, in the methods previously reported in Japan and overseas, including the methods described above, there arises problems frequently: (i) a large amount of very pure water is consumed, (ii) dialysis and ultra-filtration process, taking a long time, are included, and (iii) a large amount of urea 3 to 10 times the amount of keratin-containing substance, the raw material, or highly toxic thiourea is required.

### MEANS FOR SOLVING THE PROBLEMS

To solve the problems, the inventors have intensively studied methods for economically extracting keratin and cuticle protein from natural products containing keratin. As a result, the inventors have found that an aqueous medium solution of reduced keratin can be prepared at low cost in a short process by dipping and heating a keratin-containing substance, such as hair or wool cloth, in an aqueous medium solution containing a reducing agent and a surface active agent and then by removing water-insoluble component through filtration. In addition, the inventors found that a powder of reduced keratin can be obtained by drying (such as freeze-drying, spray-drying or the like) the solution. Furthermore, the inventors also found that since the water-insoluble component obtained in the above-mentioned filtration step primarily consists of reduced cuticle protein, an aqueous medium dispersion of the reduced cuticle protein or a dry powder thereof can be obtained by powderization of the water-insoluble component after washing.

Accordingly, the present invention provides a method for producing an aqueous medium solution of reduced keratin, an aqueous medium dispersion of reduced cuticle protein and an aqueous medium dispersion containing reduced keratin and reduced cuticle protein, characterized by heating a keratin-containing substance in an aqueous medium under the presence of a reducing agent and a surface active agent, without using a protein denaturing agent, such as urea. Furthermore, the present invention provides a method for producing a powder of reduced keratin, a powder of reduced cuticle protein or a mixed powder of reduced keratin and reduced cuticle protein, characterized by removing the solvent or dispersion medium from the aqueous medium solution of the reduced keratin, the aqueous medium dispersion of the reduced cuticle protein or the aqueous medium dispersion containing the reduced keratin and the reduced cuticle protein.

More specifically, the present invention provides:
a method (claim 1) for producing a reduced keratin solution, comprising the extraction step of dipping and heating a keratin-containing substance in an aqueous medium containing a reducing agent and a surface active agent to extract the water-soluble component contained in the keratin-containing substance into the aqueous medium, and the step of removing the water-insoluble component from the aqueous medium after the extraction step,

a method (claim 2) according to the above-mentioned method for producing the reduced keratin solution, further comprising, after the step of removing the water-insoluble component, the step of depositing and separating the water-soluble component from an aqueous medium in which the water-soluble component is dissolved, and the step of redissolving the deposited water-soluble component in the aqueous medium,

a method (claim 3) for producing a powder of reduced keratin, comprising the step of removing the solvent from the solution of reduced keratin obtained by the above-mentioned method for producing the solution of reduced keratin (claims 1 and 2),

a method (claim 4) for producing a reduced cuticle protein dispersion, comprising the extraction step of dipping and heating a keratin-containing substance in an aqueous medium containing a reducing agent and a surface active agent to extract the water-soluble component contained in the keratin-containing substance into the aqueous medium, the step of separating the water-insoluble component from the aqueous medium after the extraction step, and the step of powdering and dispersing the water-insoluble component into an aqueous medium,

a method (claim 5) for producing a powder of reduced cuticle protein by removing the dispersion medium from the reduced cuticle protein dispersion obtained by the above-mentioned method for producing the reduced cuticle protein dispersion (claim 4),

a method (claim 6) for producing an aqueous medium dispersion containing reduced keratin and reduced cuticle protein, comprising the extraction step of dipping and heating a keratin-containing substance in an aqueous medium containing a reducing agent and a surface active agent to extract the water-soluble component contained in the keratin-containing substance into the aqueous medium and the step of powderization of the water-insoluble component after the extraction step, and

a method (claim 7) for producing a mixed powder of reduced keratin and reduced cuticle protein, comprising the step of removing the solvent from the aqueous medium dispersion obtained by the above-mentioned method for producing the aqueous medium dispersion (claim 6).

Cuticle, a scale-like portion on the surface of wool or hair, is insoluble in acids, such as formic acid and acetic acid and most organic solvents (other than concentrated sulfuric acid and concentrated phosphoric acid), and not extracted. Cuticle is composed of amino acids and categorized as protein in terms of its structure (J. A. Swift, B. Rews; J. Soc. Cosmet, Chem., 27,289 (1976)). In the cosmetics industry, the scale-like portion on the surface of hair is called "cuticle" and cuticle simply means a portion in many cases. In the present specification, cuticle is described as "cuticle protein" to avoid confusion. In addition, the water-soluble component in the keratin-containing substance means a matter that is reduced using a reducing agent and can be extracted into an aqueous medium using a surface active agent, and consists primarily of reduced keratin.

Reagents and extraction/separation operations used in the methods according to the present invention and products obtained by the methods will be described below.

### [Keratin-containing substance]

The keratin-containing substance includes any substances containing keratin, a protein. Examples of keratin may preferably include hair, animal hair, such as that of sheep, yaks and cows, and feathers, such as those of chickens and ducks, and may further include nails, and horns and hoofs of domestic animals. In addition, a processed good made from the above, such as wool cloth, can also be used. In the case of wool cloth, the wool cloth is used as it is or is cut into pieces to be subjected to an extraction step. Its size should only be a size not passing through a filter, and it is not particularly required to be powdered into minute pieces.

### [Aqueous medium]

The aqueous medium may be water only or a mixture of water and a water-miscible organic solvent having water content of 50 mass % or more, preferably 80 mass % or more. Examples of water-miscible organic solvents may include lower aliphatic alcohols, such as methanol and ethanol. Examples of water may include distilled water, ultra filtration water and tap water.

### [Reducing agent]

Examples of reducing agents may include thiols, such as 2-mercaptoethanol, thioglycolic acid, cysteine, thiomalic acid, dithiothreitol and dithioerythritol, and inorganic compounds, such as sodium hydrogen sulfite. The amount of use of a reducing agent is preferably 0.01 to 0.5 mols for 10 g of a keratin-containing substance, further preferably 0.03 to 0.2 mols for 10 g of a keratin-containing substance in consideration of reaction efficiency and economical efficiency.

### [Surface active agent]

Examples of surface active agents may include anionic surface active agents, such as alkyl sulfates (e.g. sodium dodecyl sulfate), alkyl sulfate ester salts, aliphatic alcohol phosphate ester salts and sulfosuccinate ester salts;
a cationic surface active agent represented by the following formula (1):

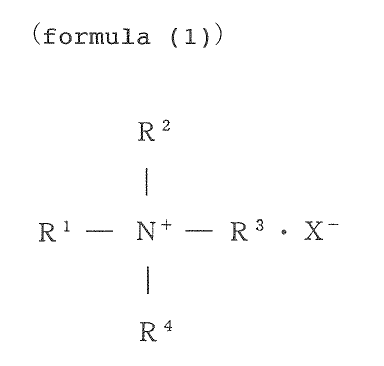

in which one or two of R¹, R², R³ and R⁴ are straight-chained or branched alkyl or hydroxyalkyl groups having 8 to 20 carbon atoms, and the remainders are hydrogen, alkyl or hydroxyalkyl having 1 to 3 carbon atoms, or benzyl groups, the nitrogen is positively charged, and X⁻ is a negatively charged ion, such as a halide ion or an alkyl sulfate ion having one or two carbon atoms;
a betaine-type ampholytic surface active agent, such as an N-carboxymethyl compound or N-sulfoalkylated compound of aliphatic amine (the hydrophobic group primarily consists of alkyl or acyl groups having 12 to 14 carbon atoms, and the counterions are alkaline metal ions); and
non-ionic surface active agents, such as a polyoxyethylene alkyl ether type, a aliphatic acid ester type, a polyethyleneimine type, a polyglycerol alkyl ether type and a polyglycerol acyl ester type (the hydrophobic group primarily consists of alkyl or acyl groups having 12 to 14 carbon atoms). The amount of such a surface active agent is preferably 5 to 50 mass % in the keratin-containing substance, more preferably 10 to 25 mass %.

### [Extraction/separation operations and products obtained thereby]

The extraction step according to the present invention is carried out, for example, by dipping a keratin-containing substance in an aqueous medium having a weight preferably 5 to 100 times, more preferably 10 to 50 times, the weight of the substance so that the substance is dipped completely, then adding a reducing agent and a surface active agent, followed by heating the mixture to extract a water-soluble component from the keratin-containing substance. The extraction of the water-soluble component is carried out by heating for 1 to 24 hours under mechanical stirring in a temperature range of 40 to 130°C or under reflux at approximately 100°C. When the heating is performed at a high temperature of 120°C or the like, the extraction is carried out under high pressure using an autoclave or the like. The pH value of the aqueous medium is in the range of neutral to weak alkaline, and the pH adjustment is performed by adding an alkaline agent or the like. It is desirable that the above-mentioned step is carried out in an atmosphere of inert gas, such as nitrogen gas. Furthermore, when the heating is performed, ultrasonic irradiation can be done instead of or in addition to the mechanical stirring. The preferable intensity of the ultrasonic irradiation varies depending on the size of the reaction system. For example, when the size of the reaction system is one liter or less, an output of 50 to 200 W is sufficient.

The reduced substance obtained in this way comprises a solution portion in which the water-soluble component extracted from the keratin-containing substance is dissolved and a water-insoluble component that is not extracted but remains. The solution portion is separated from the water-insoluble component by centrifugal separation or filtration after the extraction step. The solution portion contains reduced keratin and can be used as a reduced keratin solution as it is. However, steps of deposition, separation, washing and redissolution are usually further carried out to obtain a reduced keratin solution so that reduced keratin having higher purity can be obtained.

More specifically,
the solution portion, from which the water-insoluble component has already been separated, is acidified with diluted hydrochloric acid to pH 2.5 to 5.5, preferably pH 3.0 to 5.0, to precipitate (deposit) the reduced keratin,
then, the precipitate is removed (separated) by centrifugal separation or filtration and well washed with an aqueous medium, the pH value of which is adjusted to 3.0 to 5.5, preferably 3.5 to 5.0, containing a reducing agent of 0.05 to 0.3 mass %,
then, an appropriate amount of aqueous medium, the pH value of which is approximately neutral, containing an reducing agent of 0.05 to 0.3 mass % is added thereto, and then, a base, for example, an alkaline aqueous solution, such as ammonium water or sodium hydroxide solution, or triethylamine, is added to adjust the pH value to 8.5 to 9.5 to redissolve the mixture. The concentration of the reduced keratin in the reduced keratin solution can be adjusted as desired by changing the amount of the aqueous medium used for dissolution. Usually, the concentration is 5 to 30 mass %. When hair or wool is used as the raw material, the yield of reduced keratin is usually 25 to 43 mass % for the raw material. In the case of feathers, the yield is usually 50 to 85 mass %.

On the other hand, the water-insoluble component separated by centrifugal separation or filtration as described above contains reduced cuticle protein. The water-insoluble component containing this reduced cuticle protein is washed preferably with an aqueous medium having a pH value ranging from 3 to approximately neutral and containing a reducing agent of 0.05 to 0.3 mass %, and then treated in the aqueous medium using a Warren blender, an ultra-high speed stirring/powdering apparatus or an ultrasonic bath so as to be finely divided, whereby a dark-gray aqueous medium dispersion is obtained when the material is hair, and a milk-white aqueous medium dispersion is obtained when the material is wool. In this case, the concentration of the aqueous medium dispersion can be adjusted by changing the amount of the aqueous medium that was used for dispersion and is usually adjusted to 5 to 30 mass %. The yield of the reduced cuticle protein is usually 30 to 42 mass % for the material.

Furthermore, after the keratin-containing substance is dipped in the aqueous medium and treated with the reducing agent and the surface active agent as described above, when the water-insoluble component is powdered without separating the solution portion from the water-insoluble component, an aqueous medium dispersion containing the reduced keratin and the reduced cuticle protein can be produced. In this method, it is preferable that the water-soluble component is also subjected to the above-mentioned deposition, separation, washing and redissolution steps in order to raise purity. Still further, it is also preferable that the water-insoluble component is taken out from the aqueous medium and washed as described above.

For example, the following method can be taken as a specific method for producing the aqueous medium dispersion containing the reduced keratin and the reduced cuticle protein.

First, as described above, a keratin-containing substance is dipped in an aqueous medium, treated with a reducing agent and a surface active agent, and wholly acidified to pH 3.0 to 5.5 so that reduced keratin that has become water-insoluble is precipitated. This precipitate and the reduced cuticle protein that is a water-insoluble component mixed therewith are subjected to centrifugal separation or filtration so as to be separated from the solvent. Next, the mixture of the precipitate and the water-insoluble component is washed with an aqueous medium containing a reducing agent of 0.05 to 0.3 mass %, the pH value of which is adjusted to 3.0 to 5.5. Then, an aqueous medium having an approximately neutral pH value and containing an appropriate amount of a reducing agent of 0.05 to 0.3 mass % is added. Furthermore, a base, such as ammonium water, sodium hydroxide aqueous solution or triethylamine, is added to adjust the pH value to 8.5 to 9.5 and the mixture is treated using a Warren blender, an ultra-high speed stirring/powdering apparatus or an ultrasonic bath, whereby an aqueous medium dispersion primarily consisting of the reduced keratin and the reduced cuticle protein is obtained. In this case, the yield is usually 80 to 95 mass % for the material. The concentration of the protein (dissolved portion + dispersed portion) in the aqueous medium dispersion can be adjusted by changing the amount of the aqueous medium used for dispersion and is usually adjusted to 5 to 30 mass %.

When hair or wool was used as the keratin-containing substance and when the reduced keratin obtained as described above was subjected to amino acid analysis, it was found that approximately 5 to 10 cysteine residues and approximately 0.5 to 4 cystine residues were contained per 100 amino acid residues although the amounts of the cysteine residues and the cystine residues in the keratin may change depending on the kind of keratin-containing substance. Furthermore, by the electrophoretic analysis, it was found that the primary ingredients of the reduced keratin were proteins having molecular weights of 40,000 to 60,000 and that proteins having molecular weights of 25,000 to 30,000 were also contained in addition to the primary ingredients.

When the keratin-containing substance is feathers of birds, such as chickens or ducks, most of the keratin-containing substance is made water-soluble, and the distribution of the amino acids thereof is similar to that of the amino acids of the feathers used as the raw material. It was found that approximately 4.5 to 6 cysteine residues and approximately 1.5 to 3 cystine residues were contained per 100 amino acid residues, and the molecular weights of the proteins were approximately 5,000 to 11,000. On the other hand, the molecular weights of the proteins in the powder obtained by freeze-drying the dispersion of the reduced cuticle protein derived from hair or wool cannot be measured because the powder is insoluble. However, by an amino acid analysis, it was found that approximately 10 to 15 cysteine residues and approximately 3 to 7 cystine residues were contained per 100 amino acid residues although the analysis values had large variations.

The reduced keratin solution, the reduced cuticle protein dispersion and the dispersion containing the mixture of both can be formed into powders of the corresponding proteins respectively by removing the solvents using an ordinary drying method, such as a freeze-drying or spray-drying method. In this case, when the drying step is carried out under an atmosphere of inert gas, such as nitrogen or carbon dioxide gas, the oxidation of the SH groups can be prevented effectively. The obtained powders are stored in hermetically sealed protective containers until use. The particle diameters of the powders are in the range of 5 to 1,000 µm in many cases, and the particles having a diameter of 10 to 200 µm can be obtained under favorable conditions. The powders can be used after being redissolved in a neutral or weak alkaline aqueous medium or dispersed.

Although the mechanism for generating the reduced keratin and the reduced cuticle protein according to the present invention has not yet been clearly analyzed, it is estimated that since the disulfide bonds (S-S bonds) of a keratin-containing substance are cleaved and reduced to thiol groups (SH groups) by a substitution or reduction reaction by using a reducing agent, such as 2-mercaptoethanol, the three-dimensional cross-linking is broken and the molecular weights are lowered, and that the resulting reduced keratin is dissolved and extracted into the aqueous medium by virtue of the surface active agent used together. Although the S-S bonds of the cuticle portion are also cleaved at the same time using the reducing agent, it is assumed that since the molecules of the cuticle are also cross-linked by -CO-NH- bonds and the like, the molecular weights are not lowered completely, whereby the cuticle remains as an insoluble matter and is hardly extracted into a solution.

The method of the present invention is characterized in that a protein denaturing agent, such as urea, is not used, and that two substances, a surface active agent and a reducing agent, are used as solubilizing agents and heating is carried out under reflux or the like. With respect to yield, this method favorably compares with the method described in previous reports (Patent Documents 1 and 3 and Nonpatent Document 4) in which three substances, urea, a surface active agent and a reducing agent, are used as solubilizing agents. In addition, although the extraction treatment is carried out at a relatively high temperature, the distribution of molecular weights is not identical; this may be because no denaturing agent is used. For example, when keratin was extracted from merino wool cloth serving as a keratin-containing substance by using urea, 2-mercaptoethanol and a surface active agent at 50°C for 5 hours under a neutral condition (Nonpatent Document 4), the total amount of proteins having molecular weights of 25,000 to 30,000 measured according to a polyacrylamide electrophoresis method was approximately 10 mass % of the total weight of the extracted proteins. On the other hand, under the conditions according to the present invention (no urea; sodium dodecyl sulfate and 2-mercaptoethanol; 100°C, 5 hours; pH 7.0 to 8.5), the total amount of proteins having the same molecular weights was 30 to 40 mass %.

According to the nuclear magnetic resonance spectrum and the mass analysis of protein, the powder of the reduced keratin obtained by the method of the present invention contains the reduced keratin and a small amount of the surface active agent used for the method. However, almost all of the surface active agent can be removed by repeating washing with the aqueous medium containing the reducing agent of 0.05 to 0.3 mass % at pH 3.0 to 5.5, which is the same that used for the above-mentioned separation.

### EFFECT OF THE INVENTION

With the present invention, keratin and cuticle protein can be extracted economically as reduced types from a keratin-containing substance, such as hair, animal hair or feathers, without by using a protein denaturing agent, such as urea. These obtained products can be used as new materials. In the case that the keratin-containing substance is hair or wool, the total yield of the reduced keratin and the reduced cuticle protein is 80 mass % or more. Since these substances have numerous SH groups having high reactivity, when the substances are put in an oxidizing atmosphere, for example, being exposed to the oxygen in the air, they have a function in which S-S bonds are formed and molecules are linked together so as to become higher molecular weights. The obtained products can thus be used as the raw materials of cosmetic materials, medical materials, coating materials and surface covering materials, and can also be used as the raw materials of films, sheets, sponges, etc.

Furthermore, the mixtures of the reduced keratin solution and the aqueous medium dispersion of the reduced cuticle protein at desired mixture ratios and the mixtures of the reduced keratin powder and the reduced cuticle protein powder at desired mixture ratios can be molded into films, sheets, sponges, etc. Similarly, the reduced keratin solution and the aqueous medium dispersion of the reduced cuticle protein mixed with an aqueous solution of a synthetic macromolecule (any water-soluble macromolecule, such as polyvinyl alcohol, polyacrylic acid, and polymethylmethacrylic acid, can be used.), the pH value of which is adjusted to 7 to 10 using an alkali or acid, can be used as raw materials of films, sheets, sponges, etc., and as raw materials of cosmetic materials, medical materials, coating materials, surface covering materials, etc.

Consequently, with the present invention, hair, wool, nails, hoofs, keratin-containing products such as clothes made of wool, and the like can mostly be reused to obtain reduced keratin or reduced cuticle protein without being thrown away after use. These are used as new materials containing numerous SH groups having high reactivity to produce various products, such as medical materials, cosmetic materials, clothing materials, coating materials, electric materials, packaging materials, artificial ivories, etc.

### BEST MODES FOR CARRYING OUT THE INVENTION

Specific modes of the present invention will be described below more specifically referring to the following examples in which 2-mercaptoethanol is typically used as a reducing agent. However, the examples should not be construed to limit the scope of the present invention. In the following examples, "L" denotes liter, and "mL" denotes milliliter.

### Example 1

Totally 140 g of a wool cloth measuring 10 cm square was washed, and 1,300 mL of water, 35 g of sodium dodecyl sulfate and 35 mL of 2-mercaptoethanol were added thereto. The mixture was heated for 10 hours under reflux and gently stirring. After cooled to room temperature, the solution portion (A) was separated from the insoluble matter (B) by centrifugal separation. Washing operation was carried out two times, each comprising the step of adjusting the pH value of the solution portion (A) to approximately 3 using 1 mol/L hydrochloric acid, the step of collecting the resulting precipitate by centrifugal separation, the step of dispersing the collected precipitate in 200 mL of 0.2 mass % 2-mercaptoethanol aqueous solution at pH 3 under stirring and the step of collecting the resulting precipitate by centrifugal separation. To the precipitate obtained after the washing, 500 mL of 0.2 mass % 2-mercaptoethanol aqueous solution was added, and the pH value of the mixture was adjusted to 8.5 to 9 using a 30% ammonium aqueous solution to dissolve the resulting precipitate. Thus, approximately 500 mL of a light-brown reduced keratin solution was obtained. The concentration in this solution was obtained by protein quantitative determination of the Lowry method and found to be 9.6 mass %. After the solution was freeze-dried, the concentration obtained by the weight measurement method of dried matter of the solution was approximately 10 mass %. In addition, the yield for the raw material cloth (wool cloth) was 35%.

When the keratin powder obtained by freeze-drying the aqueous solution was subjected to the amino acid analysis method (a method in which amino acid analysis is carried out after chemical modification with iodoacetic acid), it was indicated that 7 cysteine residues and 3.5 cystine residues were contained per 100 amino acid residues. When the SH groups were quantitatively determined in terms of cysteine residues by the Ellman method, it was indicated that 7 to 8 cysteine residues were contained per 100 amino acid residues. Furthermore, when the polyacrylamide electrophoresis method was used for measurement, it was found that the keratin primarily consisted of proteins having molecular weights of 40,000 to 60,000 and that proteins having molecular weights of 25,000 to 30,000 were also contained in addition to the primary ingredients.

On the other hand, the insoluble matter (B) almost maintained the shape of the original cloth. The cloth was repeatedly washed with 0.2 mass % 2-mercaptoethanol aqueous solution at pH 3, and white reduced cuticle protein was obtained. The yield for the material cloth was 43%. To the reduced cuticle protein piece (C) of the cloth shape was added 500 mL of 0.2 mass % 2-mercaptoethanol aqueous solution, neutral in pH, and the mixture was treated using a gyratory crusher at 30,000 rpm to obtain a milky white dispersion. When the SH groups were quantitatively determined in terms of cysteine residues by the Ellman method, it was found that 12 cysteine residues were contained per 100 amino acid residues in the substance that was contained in the dispersion.

The reduced keratin solution and the aqueous medium dispersion of the reduced cuticle protein described above were respectively freeze-dried to obtain powders. The powder of the reduced keratin was redissolved in a 0.2 mass % 2-mercaptoethanol aqueous solution, pH 8.5, to give a solution.

When 0.1 g of glycerol was added to 10 mL of the reduced keratin solution described above and the mixture was cast onto the flat surface of a PET film and dried, a transparent and flexible film was obtained. The tensile strength (at 62% relative humidity) of the film after 15 minutes of drying at 80°C was 37 MPa. On the other hand, the reduced cuticle protein dispersion gave a semi-transparent film using a similar casting method. The tensile strength (at 62% relative humidity) of the film after 15 minutes of drying at 80°C was 4 MPa. In addition, the film obtained from the mixture of 5 mL of the reduced keratin solution and 5 mL of the aqueous medium dispersion of the reduced cuticle protein using a casting method similar to that described above flexible although no glycerol added. The tensile strength (at 62% relative humidity) of the film after 15 minutes of drying at 80°C was 47 MPa, higher than those of the films described above.

Furthermore, since the reduced cuticle protein piece (C) described above has numerous SH groups, S-S bonds are formed by oxidization and the pieces strongly stick each other. For example, a stable cylinder, the shape of which was maintained even in water of 100°C, was obtained by washing and then winding the reduced cuticle protein piece (C) around a cylinder (3 cm in diameter), by overlapping the ends thereof and by drying the obtained cylinder.

Moreover, the reduced cuticle protein piece (C) is very effective as a heavy metal ion trapping agent by virtue of the function of the SH groups. For example, when 50 mg of the reduced cuticle protein piece (C) was dipped in 30 mL of an aqueous mercuric chloride solution, in which pH is 3.5 and each Hg concentration is 10, 20, 50 or 100 mg/L, and stirred for 30 minutes at room temperature, and Hg remaining in the aqueous solution was quantitatively determined using the atomic absorption spectrometry, the Hg concentrations after the stirring were 0.1, 0.8, 3.6 and 9 mg/L, respectively. When the Hg concentrations were measured in the same conditions except that a wool cloth, the raw material, was used instead of the reduced cuticle protein piece (C), the Hg concentrations were 1.5, 5.9, 40 and 80 mg/L, respectively. According to this comparison experiment, it is obvious that the reduced cuticle protein piece (C) has an excellent capacity of adsorbing heavy metal ions, such as mercury ions.

### Example 2

Totally 140 g of a wool cloth measuring 10 cm square was washed, and 1,300 mL of water, 35 g of sodium dodecyl sulfate and 35 mL of 2-mercaptoethanol were added thereto. The mixture was heated for 10 hours under reflux and stirring. After cooled to room temperature, the pH value of the mixture was adjusted to 3, and the resulting precipitate was collected by centrifugal separation. The precipitate was subjected to washing operation three times, each comprising the step of stirring and dispersing the precipitate in 0.2 mass % 2-mercaptoethanol aqueous solution at pH 3 and the step of collecting the resulting precipitate by centrifugal separation. To the precipitate obtained after the washing, 1,000 mL of 0.2 mass % 2-mercaptoethanol aqueous solution was added, and the pH value of the mixture was adjusted to 8.5 to 9 using a 30% ammonium aqueous solution. Then, the mixture was crushed using an ultra-high speed crusher (30,000 rpm) to obtain approximately 1,000 mL of a light-brown dispersion containing reduced keratin and reduced cuticle protein.

The protein concentration in the dispersion was approximately 12 mass % that was obtained on the basis of the mass of the powder obtained by freeze-drying the dispersion. When the powder was subjected to the amino acid analysis method (a method in which amino acid analysis is carried out after chemical modification with iodoacetic acid), it was indicated that 8 cysteine residues and 3 cystine residues were contained per 100 amino acid residues, and the distribution of the amino acids other than the cysteine and cystine residues was nearly identical with the amino acid distribution of the wool cloth. Polyacrylamide electrophoresis for the powder showed protein bands identical with the reduced keratin obtained in Example 1 and a band remained at original point, and the band at original point indicates higher molecular weight protein. Hence, it was indicated that the powder was formed of reduced keratin and reduced cuticle protein containing numerous SH groups.

### Example 3

A mixture of 100 g of washed chicken feathers, 1,000 mL of water, 20 g of sodium dodecyl sulfate and 20 mL of 2-mercaptoethanol was heated for 9 hours under reflux and stirring. After cooled to room temperature, the solution portion was separated from the insoluble matter by centrifugal separation. The pH value of the solution portion was adjusted to approximately 3 to 4 using 1 mol/L hydrochloric acid, and the resulting precipitate was collected by centrifugal separation. After removing the precipitate, methanol was gradually added to the solution and the resulting precipitate was collected by centrifugal separation. Washing operation was carried out two times, each comprising the step of combining these precipitates, the step of dispersing the precipitates in 200 mL of 0.2 mass % 2-mercaptoethanol aqueous solution at pH 3 under stirring and the step of collecting the resulting precipitate by centrifugal separation. To the precipitate after washing, 600 mL of 0.2 mass % 2-mercaptoethanol aqueous solution was added, the pH value of the mixture was adjusted to 8.5 using a 30% ammonium aqueous solution to dissolve the precipitate and 650 mL of a light-brown reduced keratin solution was obtained. The concentration in this solution was obtained by protein quantitative determination by the Lowry method and found to be 12 mass %. After the solution was freeze-dried, the yield obtained by weight measurement was approximately 11 mass %. Furthermore, when the keratin powder obtained by freeze-drying the aqueous solution was subjected to the amino acid analysis method, it was found that 5.1 cysteine residues and 2.1 cystine residues were contained per 100 amino acid residues. Moreover, by the polyacrylamide electrophoresis method, it was found that the keratin primarily consisted of proteins having molecular weights of 5,000 to 11,000.

### Example 4

To 100 g of powdered cow horn pieces were added 1,500 mL of water, 30 g of sodium dodecyl sulfate and 30 mL of 2-mercaptoethanol. The mixture was heated for 24 hours under reflux and stirring. After cooled to room temperature, the solution portion was separated from the insoluble matter by centrifugal separation. Washing operation was carried out two times, each comprising the step of adjusting the pH value of the solution portion to approximately 3 using 1 mol/L hydrochloric acid, the step of collecting the resulting precipitate by centrifugal separation, the step of dispersing the precipitate in 200 mL of 0.2 mass % 2-mercaptoethanol aqueous solution at pH 3 under stirring and the step of collecting the resulting precipitate by centrifugal separation. To the precipitate after washing, 500 mL of 0.2 mass % 2-mercaptoethanol aqueous solution was added, and the pH value of the mixture was adjusted to pH 8.5 using a 30% ammonium aqueous solution, and the resulting precipitate was dissolved to obtain approximately 500 mL of a dark-brown reduced keratin solution. The solution thus obtained was freeze-dried. The yield obtained by weight measurement was approximately 60 mass % for the material horn.

In addition, when the reduced keratin powder (D) obtained by freeze-drying the solution was subjected to the amino acid analysis method, it was found that 4 cysteine residues and 3 cystine residues were contained per 100 amino acid residues. On the other hand, the insoluble matter maintained the shape of the original horn pieces. After the horn pieces were repeatedly washed with 0.2 mass % 2-mercaptoethanol aqueous solution at pH 3, 500 mL of 0.2 mass % 2-mercaptoethanol aqueous solution was added therto, and the mixture was treated using a gyratory crusher at 30,000 rpm to obtain a dark brown dispersion. The dispersion was freeze-dried to obtain powder (E). After the powder (D) was mixed with the weight of the powder (E), the mixture was subjected to compression molding (at a pressure of 5 to 10 kg/cm²) under heating at 160°C so as to be formed into a sheet shape, and a sheet having a high strength was obtained.

### Example 5

The reduced keratin powder and the reduced cuticle protein powder, prepared in Example 1, and an ethanol-water mixture (mass ratio 9:1) were mixed well at a mass ratio of 20:10:30. The mixture was subjected to compression molding under heating at from 70 to 160°C using a stainless steel mold measuring 5 by 5 cm and 0.3 cm in depth, and a light-yellow semi-transparent sheet was obtained. The bending strength and the tensile strength of this sheet was 130 to 150 MPa and 50 to 95 MPa, respectively, and the sheet was able to withstand mechanical impacts and was almost free from swelling in water at 50°C, thereby being excellent in water resistance.

## Claims

1. A method for producing a reduced keratin solution, comprising the extraction step of dipping and heating a keratin-containing substance in an aqueous medium containing a reducing agent and a surface active agent to extract the water-soluble component contained in the keratin-containing substance into the aqueous medium, and the step of removing the water-insoluble component from the aqueous medium after the extraction step.

2. A method for producing the reduced keratin solution according to claim 1, further comprising the step of depositing and separating the water-soluble component from an aqueous medium in which the water-soluble component is dissolved, after the step of removing the water-insoluble component, and the step of redissolving the deposited water-soluble component in the aqueous medium.

3. A method for producing a powder of reduced keratin, comprising the step of removing the solvent from the solution of reduced keratin obtained by the method for producing the solution of reduced keratin according to claim 1 or 2.

4. A method for producing a reduced cuticle protein dispersion, comprising the extraction step of dipping and heating a keratin-containing substance in an aqueous medium containing a reducing agent and a surface active agent to extract the water-soluble component contained in the keratin-containing substance into the aqueous medium, the step of separating the water-insoluble component from the aqueous medium after the extraction step, and the step of powdering and dispersing the water-insoluble component into an aqueous medium.

5. A method for producing a powder of reduced cuticle protein by removing the dispersion medium from the reduced cuticle protein dispersion obtained by the method for producing the reduced cuticle protein dispersion according to claim 4.

6. A method for producing an aqueous medium dispersion containing reduced keratin and reduced cuticle protein, comprising the extraction step of dipping and heating a keratin-containing substance in an aqueous medium containing a reducing agent and a surface active agent to extract the water-soluble component contained in the keratin-containing substance into the aqueous medium, and the step of powdering the water-insoluble component after the extraction step.

7. A method for producing a mixed powder of reduced keratin and reduced cuticle protein, comprising the step of removing the solvent from the aqueous medium dispersion obtained by the method for producing the aqueous medium dispersion according to claim 6.
